# DEMANDE DE BREVET EUROPEEN

(11) **EP 3 831 802 A2**
(43) Date de publication de la demande: **09.06.2021**
(21) Numéro de dépôt: 20215854.9
(22) Date de dépôt: 20.01.2014
(51) Int. Cl.: C07C 45/82, C07C 47/58, B01D 3/14

(54) **PROCEDE DE PURIFICATION DE LA VANILLINE NATURELLE**

(30) Priorité: 24.01.2013 FR 1350617; 23.12.2013 FR 1363444
(62) Demande divisionnaire de: 14712214.7
(71) Demandeur: RHODIA OPERATIONS, 93300 Aubervilliers (FR)
(72) Inventeur: GAYET, Hubert, 69800 SAINT-PRIEST (FR); REVELANT, Denis, 69740 GENAS (FR); VIBERT, Martine, 69006 LYON (FR)
(74) Mandataire: Jacques, Marie-Caroline Anne

(57) **Abrégé**

Procédé de purification de vanilline naturelle comprenant au moins une étape dans laquelle on évapore de la vanilline naturelle.

## Description

La présente invention concerne un procédé de purification de la vanilline naturelle. L'invention concerne également de la vanilline naturelle qui peut être obtenue par le procédé selon l'invention et une installation pour produire de la vanilline naturelle purifiée..

La vanilline peut être obtenue par deux voies distinctes :
- une voie dite naturelle basée sur un procédé biotechnologique comprenant notamment la culture d'un microorganisme (champignons, bactéries, etc.) apte à permettre la biotransformation d'un substrat de fermentation en vanilline. Il est notamment connu de EP0885968 et de EP0761817 un tel procédé dans lequel le substrat de fermentation est l'acide férulique. Ce procédé aboutit à la préparation d'une vanilline dite vanilline naturelle ;
- une voie dite synthétique comprenant des réactions chimiques classiques à partir du gaïacol ne faisant pas intervenir de microorganisme. Ce procédé aboutit à la préparation d'une vanilline dite vanilline synthétique.

Actuellement, la vanilline naturelle, obtenue par la voie dite naturelle, est purifiée par extraction par un solvant suivie d'une cristallisation. Ce procédé conduit à une vanilline en limite de spécification : pureté moyenne, colorée, et à un rendement de l'ordre de 90%. L'étape de cristallisation présente l'inconvénient de nécessiter l'utilisation de solvants organiques. L'utilisation de tels solvants, bien qu'autorisée, peut présenter des inconvénients d'un point de vue toxicologie et/ou environnemental.

Or, actuellement, personne n'est capable de fournir un procédé de purification de la vanilline naturelle ne nécessitant pas l'utilisation de solvant organique de cristallisation.

Il est connu des procédés de purification de la vanilline synthétique par distillation. Cependant, les flux en fin de procédé de préparation de la vanilline par voie naturelle ou par voie synthétique sont bien distincts. Le flux en sortie de procédé de préparation de la vanilline naturelle comprend typiquement de la vanilline naturelle, de l'alcool vanillique et un solvant, de préférence un solvant alimentaire par exemple l'acétate d'éthyle. Ce flux peut également comprendre de l'acide vanillique, de l'acide férulique, du gaïacol, de l'acide benzoïque, des dimères et des trimères, des dérivés du gaïacol et de l'eau. Le flux en sortie de procédé de préparation de la vanilline synthétique comprend typiquement de la vanilline synthétique, de l'ortho-vanilline, des trimères, du gaïacol et du DFG (Diformaldéhyde gaiacol). La manière d'appréhender ces différents flux est donc distincte.

Un objectif de la présente invention est donc de fournir un procédé de purification de la vanilline naturelle en particulier ne nécessitant pas l'utilisation de solvant.

Un autre objectif de la présente invention est de fournir un procédé de purification de la vanilline naturelle qui soit de mise en œuvre simple et qui puisse être mis en œuvre de façon continue pour être compatible avec des procédés industriels. En particulier, la présente invention recherche la mise à disposition d'un procédé de purification de la vanilline naturelle conduisant à un rendement supérieur à 90%, de préférence supérieur à 95%.

Un autre objectif encore de la présente invention est de fournir un procédé de purification de la vanilline naturelle modulable permettant d'obtenir, selon les spécifications recherchées :
- une vanilline naturelle très pure, par exemple d'une pureté supérieure ou égale à 99% ; ou
- une vanilline naturelle de pureté moindre, par exemple de pureté de 96 à 98,9% et possédant, de part la présence de certaines impuretés, des propriétés organoleptiques différentes de celles de la vanilline naturelle très pure et qui se rapprochent des propriétés organoleptiques de la vanille gousse. Le procédé selon l'invention permet donc avantageusement de maîtriser le taux d'impuretés résiduelles et notamment celles ayant une influence positive sur la qualité organoleptique de la vanilline naturelle finale.
La vanilline naturelle étant un produit thermosensible (notamment formation de trimères par chauffage de la vanilline), un autre objectif de la présente invention est de fournir un procédé de purification qui conserve toutes les propriétés de la vanilline.

Il serait en outre avantageux de fournir un procédé de purification de vanilline naturelle plus respectueux de l'environnement et permettant d'obtenir une vanilline naturelle très pure et très faiblement colorée sans dégradation de ses propriétés. D'autres objectifs encore apparaîtront à la lecture de la description de l'invention qui suit.

La vanilline naturelle selon l'invention, et obtenue par le procédé de l'invention, est une substance aromatisante naturelle selon l'article 9.2.c) du règlement CE1334/2008. C'est-à-dire une substance aromatisante obtenue par des procédés physiques, enzymatiques ou microbiologiques à partir de matières d'origine végétale, animale ou microbiologique prises en l'état ou après leur transformation pour la consommation humaine par un ou plusieurs des procédés traditionnels de préparation des denrées alimentaires. Une substance aromatisante naturelle correspond à une substance qui est naturellement présente et a été identifiée dans la nature.
L'invention concerne donc un procédé de purification de vanilline naturelle comprenant au moins une étape dans laquelle on évapore de la vanilline naturelle.

La Demanderesse a découvert, de manière surprenante, qu'un nouveau procédé de purification de la vanilline naturelle permettait d'obtenir un produit de pureté supérieure ou égale à 99% en poids et présentant une couleur inférieure ou égale à 200 Hazen en solution éthanolique à 10% en poids.

Ce procédé permet, en particulier lorsqu'il est appliqué à une vanilline naturelle de départ ayant un degré de pureté allant de 95 à 99% en poids, d'obtenir une vanilline très faiblement colorée avec un rendement supérieur ou égal à 95% en poids par rapport à la quantité initiale de vanilline présente dans le produit de départ.
Dans le cadre de la présente invention, on entend par « procédé de production de la vanilline naturelle» des procédés de préparation de la vanilline par des voies biotechnologiques. De tels procédés comprennent notamment la culture d'un microorganisme apte à permettre la biotransformation d'un substrat de fermentation en vanilline. Il est notamment connu de EP0885968 et EP0761817 un tel procédé dans lequel le substrat de fermentation est l'acide férulique.

Dans le cadre de la présente invention, on entend par « vanilline naturelle » la vanilline obtenue par des procédés de préparation tels que définis ci-dessus.

Dans le cadre de la présente invention, on entend par « impuretés ultra-légères » ou les « ultra-légers » des composés dont la volatilité est plus élevée que celle de la vanilline naturelle dans les conditions de pression et de température considérées. Le rapport entre la tension de vapeur des ultra-légers et la tension de vapeur de la vanilline est compris entre 60 et 200 à 134°C (ou à la température de distillation). Parmi les impuretés ultra-légères on peut citer l'acétate d'éthyle.
On entend par « impuretés légères » ou les « légers » des composés dont la volatilité relative est intermédiaire entre celle des ultra-légers et celle de la vanilline naturelle dans les conditions de pression et de température considérées. Parmi les impuretés légères ont peut citer l'acide benzoïque et le gaïacol.

On entend par « impuretés lourdes » ou les « lourds » des composés dont la volatilité relative est plus faible que celle de la vanilline naturelle dans les conditions de pression et de température considérées. Le rapport entre la tension de vapeur des lourds et la tension de vapeur de la vanilline est compris entre 0,3 et 0,7 à 150°C (ou à la température de distillation). Parmi les impuretés lourdes on peut citer les dimères, les trimères de la vanilline (c'est-à-dire des composés présentant un squelette ayant deux respectivement trois groupements phényles, les dimères étant avantageusement choisis parmi les diphényleméthanes), l'alcool vanillique, l'acide vanillique, l'acide férulique, les benzoates, notamment le benzoate de sodium, les dérivés du gaïacol, par exemple le 4-méthyle gaïacol et le 4-éthyle gaïacol.
De manière générale, la volatilité est la capacité d'une substance à se vaporiser.

Dans un premier aspect, la présente invention concerne un procédé de purification d'un flux issu d'un procédé de production de vanilline naturelle, ledit flux comprenant de la vanilline naturelle et au moins de l'alcool vanillique, par distillation permettant la séparation de la vanilline naturelle de l'alcool vanillique.

En fin de procédé de préparation de la vanilline naturelle on obtient un flux liquide *(F1)* comprenant la vanilline naturelle, les ultra-légers, les légers et les lourds. Dans un mode de réalisation particulier les ultra-légers représentent de 50 à 90% en poids du flux *(F1),* les légers représentent de 0,1 à 10% en poids du flux *(F1),* les lourds représentent de 0,1 à 15% en poids du flux *(F1),* la vanilline naturelle représente de 5 à 35% en poids du flux *(F1).* De préférence, les ultra-légers représentent de 60 à 80% en poids du flux *(F1),* les légers représentent de 0,5 à 5% en poids du flux *(F1),* les lourds représentent de 1 à 10% en poids du flux *(F1),* la vanilline naturelle représente de 10 à 30% en poids du flux *(F1).*

La composition du flux *(F1)* de départ est donnée à titre indicatif et le procédé de l'invention convient également pour des mélanges comprenant plus ou moins de vanilline naturelle.

Dans un mode de réalisation particulier, le flux *(F1)* comprend :
- de 5 à 35% en poids de vanilline naturelle, notamment de 10 à 30 % en poids ;
- de 0,05 à 10 % en poids d'alcool vanillique, notamment de 0,1 à 5 % en poids;
- de 0,0 à 2 % en poids d'acide vanillique, notamment de 0,01 à 0,6 % en poids ;
- de 0,0 à 2 % en poids d'acide férulique, notamment de 0,01 à 0,9 % en poids ;
- de 0,0 à 2 % en poids de gaïacol, notamment de 0,01 à 0.6 %en poids ;
- de 0,0 à 5% en poids de dimères et de trimères, de préférence de 0,05 à 5 % en poids de dimères et de trimères, notamment de 0,1 à 5% en poids ; les dimères et trimères étant des composés présentant un squelette ayant respectivement deux ou trois groupements phényles, les dimères étant avantageusement choisis parmi les diphényleméthanes ;
- de 0,0 à 5% en poids de divers composés organiques également biosynthétisés (comme par exemple 4-méthyle gaïacol, 4-éthyle gaïacol et/ou 4-vinyle gaïacol), de préférence de 0,01 à 5 % en poids, notamment de 0,05 à 1% en poids ;
- de 0,0 à 10% en poids d'acide benzoïque, de préférence de 0,01 à 10 % en poids d'acide benzoïque, notamment de 0,05 à 5% en poids ;
- de 0,0 à 5% en poids d'eau, de préférence de 0,01 à 5 % en poids d'eau, notamment de 0,05 à 3 % en poids ;
- le complément à 100% en poids étant un solvant alimentaire par exemple acétate d'éthyle.

De préférence, la pression de service lors de la distillation est comprise entre 2 et 10 mbar, de préférence entre 3 et 6 mbar, par exemple elle est de 4 mbar.

Dans le cadre de la présente invention on entend par « pression de service » la pression en tête de la colonne de distillation.

De façon avantageuse, la perte de charge dans la colonne de distillation doit être la plus faible possible. De préférence, la perte de charge est comprise entre 1 et 10 mbar, de préférence entre 2 et 8 mbar, par exemple la perte de charge est de 3 ou 4 mbar.

Dans le cadre de la présente invention, et sauf indication contraire, l'expression « compris entre x et y » inclue les valeurs x et y. Dans le cadre de l'invention cette expression signifie également « de x à y ».

Dans le procédé selon l'invention, une ou plusieurs colonnes de distillation sont utilisées.

De préférence, la ou les colonnes de distillation comprennent de 7 à 40 plateaux théoriques, de préférence de 12 à 30 plateaux théoriques.

De façon avantageuse, le procédé selon l'invention permet d'obtenir une vanilline naturelle de pureté supérieure ou égale à 96%, de préférence supérieure ou égale à 98%. Dans un mode de réalisation, le procédé selon l'invention permet d'obtenir une vanilline naturelle très pure, notamment d'une pureté supérieure ou égale à 99% et contenant de préférence moins de 1000 ppm d'alcool vanillique. Ce mode de réalisation peut être obtenu en adaptant les paramètres de distillation.

Dans un autre mode de réalisation, le procédé selon l'invention permet d'obtenir une vanilline naturelle de pureté moindre, par exemple de pureté de 96 à 98,9% et comprenant des impuretés conférant au flux de vanilline naturelle obtenu des propriétés organoleptiques différentes de celle de la vanilline naturelle très pure et proches de celles de la vanille gousse. Dans ce mode de réalisation, le flux final de vanilline naturelle peut comprendre jusqu'à 3% en poids d'impuretés. Parmi les impuretés on retrouve en majorité l'alcool vanillique, et les dimères et/ou trimères, notamment les diphényleméthanes, qui peuvent représenter jusqu'à 1% en poids du flux, et en minorité l'acide vanillique, le gaïacol, l'acide benzoïque et les composés organiques biosynthétisés, qui peuvent être présents chacun jusqu'à 500 ppm dans le flux. Ce mode de réalisation peut être obtenu en adaptant les paramètres de distillation.

Dans le cadre du procédé de l'invention, il peut se produire une réaction chimique de trimérisation de la vanilline qui affecte le rendement en vanilline.

Le procédé de purification de la vanilline naturelle par distillation selon la présente invention peut être mené de différente manière :
- par distillation discontinue ;
- par distillation continue ; ou
- par distillation continue sur colonne à cloison (« Divided Wall Column » - DWC).

### Distillation discontinue

Dans un mode de réalisation, le procédé selon l'invention est mené par distillation discontinue. Le procédé comprend alors différentes opérations unitaires de distillation permettant la séparation de manière distincte des ultra-légers, des légers, de la vanilline et des lourds.

Dans le procédé discontinu, le flux *(F1)* est chargé dans une colonne de distillation. Le procédé permet de récupérer :
- dans une première fraction les ultra-légers ;
- dans une seconde fraction les légers pouvant éventuellement comprendre de la vanilline naturelle à une faible concentration ;
- dans une troisième fraction le flux comprenant la vanilline naturelle ; et
- dans le culot de distillation les lourds pouvant éventuellement comprendre de la vanilline naturelle à une faible concentration.

Dans ce mode de réalisation, on entend par « faible concentration » une concentration de préférence d'au plus 10%, de préférence d'au plus 5%, par exemple d'au plus 3% en poids par rapport au poids total de la fraction.

Les première, seconde et troisième fractions peuvent avantageusement être récupérées par condensation mettant en œuvre des dispositifs classiques connus de l'homme du métier.

L'homme du métier est parfaitement en mesure de fixer les paramètres de distillation tels que notamment le diamètre de la colonne, la hauteur de colonne et le garnissage. On rappellera simplement ce qui suit. La taille (notamment le diamètre) des colonnes de distillation dépend du flux circulant et de la pression interne. Leur dimensionnement se fera donc principalement suivant le débit de mélange à traiter. On précisera que la colonne peut être garnie indifféremment de plateaux ou de garnissage ordonné ou tissé, comme cela est parfaitement connu de l'homme du métier. L'installation étant déterminée, l'homme du métier ajuste les paramètres de fonctionnement de la colonne.

De préférence, la colonne de distillation comprend de 7 à 30 plateaux théoriques, de préférence de 5 à 15 plateaux théoriques, par exemple 7 à 10 plateaux théoriques.

De préférence, le garnissage de la colonne est un garnissage tissé de surface de 400 à 600 m²/m³.

Ce procédé de distillation permet d'obtenir la vanilline naturelle avec un rendement de 90% à 95 %, de préférence de 90% à 93%.

Dans un mode de réalisation, le procédé selon l'invention permet d'obtenir une vanilline naturelle très pure, notamment d'une pureté supérieure ou égale à 99% et contenant de préférence moins de 1000 ppm d'alcool vanillique.

Dans un autre mode de réalisation, le procédé selon l'invention permet d'obtenir une vanilline naturelle de pureté moindre, par exemple de pureté de 96 à 98,9% et comprenant des impuretés conférant au flux de vanilline naturelle obtenu des propriétés organoleptiques différentes de celle de la vanilline naturelle très pure et proches de celles de la vanille gousse. Dans ce mode de réalisation, le flux final de vanilline naturelle peut comprendre jusqu'à 3% en poids d'impuretés. Parmi les impuretés on retrouve en majorité l'alcool vanillique, et les dimères et/ou trimères, notamment les diphényleméthanes, qui peuvent représenter jusqu'à 1% en poids du flux, et en minorité l'acide vanillique, le gaïacol, l'acide benzoïque et les composés organiques biosynthétisés, qui peuvent être présents chacun jusqu'à 500 ppm dans le flux.

### Distillation continue

Dans un mode de réalisation, le procédé selon l'invention est mené par distillation continue.

### ■ Etêtage/equeutage

Dans un mode de réalisation particulier le procédé mené par distillation continue comprend les étapes suivantes :
a) dans une première étape, le flux *(F1)* est traité afin d'éliminer les ultra-légers (flux *(F11))* qu'il contient et plus particulièrement l'acétate d'éthyle, le flux ainsi obtenu comprenant la vanilline naturelle, les légers et les lourds est appelé flux *(F2) ;*
b) dans une seconde étape, le flux *(F2)* est alimenté dans une première colonne de distillation, permettant de récupérer en tête de distillation un flux *(F12)* comprenant les légers et pouvant éventuellement comprendre une faible concentration de vanilline naturelle et en pied de distillation un flux *(F13)* comprenant la vanilline naturelle et les lourds et pouvant éventuellement comprendre une faible concentration de légers ;
c) le flux *(F13)* est alimenté de façon continue dans une seconde colonne de distillation permettant de récupérer en tête de distillation un flux *(F14)* comprenant la vanilline naturelle et en pied de distillation un flux *(F15)* comprenant les lourds et éventuellement une faible concentration en vanilline naturelle.

L'homme du métier est parfaitement en mesure de fixer les paramètres de distillation tels que notamment le diamètre de la colonne, la hauteur de colonne et le garnissage. On rappellera simplement ce qui suit. La taille (notamment le diamètre) des colonnes de distillation dépend du flux circulant et de la pression interne. Leur dimensionnement se fera donc principalement suivant le débit de mélange à traiter. On précisera que la colonne peut être garnie indifféremment de plateaux ou de garnissage ordonné ou tissé, comme cela est parfaitement connu de l'homme du métier. L'installation étant déterminée, l'homme du métier ajuste les paramètres de fonctionnement de la colonne.

Dans ce mode de réalisation, on entend par « faible concentration » une concentration de préférence d'au plus 5%, de préférence d'au plus 3%, par exemple d'au plus 2% en poids par rapport au poids total de la fraction.

Selon un mode de réalisation préféré du schéma étêtage/équeutage, une partie au moins du flux *(F15)* est mélangé au flux *(F14)* de manière à pouvoir faire varier la composition finale d'un flux de vanilline naturelle purifiée.

De façon préférée, le flux *(F12)* en tête de distillation à l'étape b) comprend les légers, et notamment l'acide benzoïque, le gaïacol et éventuellement une faible concentration en vanilline naturelle.

De préférence, la colonne de distillation de l'étape b) comprend de 20 à 35 plateaux théoriques, de préférence de 23 à 30 plateaux théoriques. La colonne de distillation de l'étape b) comprend notamment de 8 à 15 plateaux théoriques pour la section de concentration et de 12 à 20 plateaux théoriques pour la section d'épuisement, de préférence de 10 à 13 plateaux théoriques pour la section de concentration et de 13 à 17 plateaux théoriques pour la section d'épuisement.
La colonne de distillation de l'étape b) peut être une colonne de distillation équipée d'un garnissage ordonné sur une hauteur de 2x2600 mm à 2x3000 mm (2600mm à 3000 mm pour chacune des sections de concentration et d'épuisement). Le garnissage de la colonne est de préférence un garnissage tissé du fait des très basses pressions de mise en œuvre.
Dans la colonne de l'étape b), la pression de service est de préférence comprise entre 2 et 10 mbar, de préférence entre 3 et 6 mbar. La perte de charge est de préférence de 1 à 10 mbar, de préférence de 1 à 8 mbar. Lors de l'étape b) le taux de reflux est compris entre 10 et 30, de préférence entre 15 et 25.

Le taux de reflux est défini par le rapport du débit de matière réinjectée de la tête de la colonne vers l'intérieur de la colonne (soit le débit de reflux) et du débit sortant effectivement à la sortie de la tête de colonne.

Le point d'alimentation du flux *(F2)* est choisi de façon connue par l'homme du métier, il est notamment choisi de façon à éviter les rétro-mélanges. De préférence, le point d'alimentation se trouve en haut de la section d'épuisement.

Le ratio du débit de distillation (débit du flux *(F12)*) sur le débit d'alimentation (débit du flux (F2)) est de préférence de 0,05 à 0,5.

Le débit de reflux est de préférence de 1,4 à 3 fois le débit d'alimentation, par exemple il est de 2 fois le débit d'alimentation.

De préférence au cours de l'étape b), la température de pied de colonne est comprise entre 100°C et 160°C, notamment entre 120°C et 150°C. De préférence au cours de l'étape b) la température en tête de colonne est comprise entre 80°C et 120°C, de préférence entre 90°C et 110°C.

Le temps de séjour de la vanilline en pied de la colonne de l'étape b) est compris entre 30 minutes et 5 heures, il est par exemple de 3 heures.

La distillation est démarrée en ajustant le débit de flux *(F12)* pour conserver une température sensible de préférence de 100 à 160 °C. L'écart de température entre la tête (température vapeurs) et le pied (température du liquide) est de préférence compris entre 35 et 45°C.

Pour effectuer la distillation de l'étape b) l'apport des calories en pied de colonne peut être fait notamment par un bouilleur à film tombant ou à film raclé, par un échangeur tubes-calandre chauffé à la vapeur ou par un fluide caloporteur, par l'intermédiaire de serpentins de chauffage alimentés à la vapeur ou par un fluide caloporteur ou par tout autre dispositif équivalent, de préférence par un fluide caloporteur. De préférence l'énergie pour la distillation est apportée par un bouilleur de type à film tombant, un tubes-et-calandre à circulation forcée ou un tubes-et-calandre à thermosiphon.

Le flux *(F13)* de pied de colonne en fin d'étape b) est envoyé vers la colonne de distillation de l'étape c) appelée également colonne d'équeutage.

De préférence, la colonne de l'étape c) comprend de 10 à 30 plateaux théoriques, de préférence de 15 à 25 plateaux théoriques. La colonne de distillation de l'étape c) comprend notamment de 4 à 14 plateaux théoriques pour la section d'épuisement et de 6 à 16 plateaux théoriques pour la section de concentration.

De préférence la colonne de l'étape c) est équipée par exemple d'un garnissage ordonné tissé. Le point d'entrée du flux *(F13)* se situe entre le cinquième et le quinzième plateau théorique en partant du bas de la colonne de distillation.

Dans la colonne de l'étape c), la pression de service est de préférence comprise entre 2 et 10 mbar, de préférence entre 3 et 6 mbar, par exemple 4 mbar. La perte de charge est de préférence de 1 à 10 mbar, de préférence de 1 à 8 mbar, par exemple elle est de 4 mbar. Lors de l'étape c) le taux de reflux est compris entre 2 et 10, de préférence entre 2,5 et 6, il est par exemple de 3.

Le ratio du débit de distillation (débit du flux *(F14)*) sur le débit d'alimentation (débit du flux *(F2)*) est de préférence de 0,5 à 0,95.

Le débit de reflux est de préférence de 1,4 à 3 fois le débit d'alimentation, par exemple il est de 2 fois le débit d'alimentation.

De préférence :
- le débit du flux *(F1)* est de 150 à 220 kg/h ;
- le débit du flux *(F11)* est de 100 à 180 kg/h ;
- le débit du flux *(F2)* est de 40 à 55 kg/h ;
- le débit du flux *(F12)* est de 3 à 6 kg/h ;
- le débit du flux *(F13)* est de 34 à 52 kg/h ;
- le débit du flux *(F14)* est de 20 à 60 kg/h.

De préférence au cours de l'étape c), la température de pied de colonne est comprise entre 160°C et 200°C, notamment entre 170°C et 190°C. De préférence au cours de l'étape c) la température en tête de colonne est comprise entre 100°C et 160°C, de préférence entre 110°C et 140°C.

Le temps de séjour de la vanilline en pied de colonne de l'étape c) est compris entre 15 minutes et 15 heures, de préférence entre 30 minutes et 15 heures, de manière plus préférée entre 7 et 15 heures. Afin de réduire ce temps de séjour entre 15 minutes et 8 heures et limiter la dégradation de la vanilline naturelle, qui est thermosensible, il peut être intéressant d'ajouter en pied de colonne un corps mort. Les corps morts sont bien connus de l'homme du métier, ils peuvent notamment consister en des billes de verre.

Pour effectuer la distillation de l'étape c) l'apport des calories en pied de colonne peut être fait notamment par un bouilleur à film tombant ou à film raclé, par un échangeur tubes-calandre chauffé à la vapeur ou par un fluide caloporteur, par l'intermédiaire de serpentins de chauffage alimentés à la vapeur ou par un fluide caloporteur ou par tout autre dispositif équivalent, de préférence par un fluide caloporteur. De préférence, l'énergie pour la distillation est apportée par un tubes-et-calandre à circulation forcée ou un échangeur raclé.

Dans un mode de réalisation, le procédé selon l'invention permet d'obtenir une vanilline naturelle très pure, notamment d'une pureté supérieure ou égale à 99% et contenant de préférence moins de 1000 ppm d'alcool vanillique. Ce mode de réalisation peut être obtenu en adaptant les paramètres de distillation, notamment le ratio du débit de distillation *(F14)* et du débit d'alimentation *(F2)* est alors de préférence compris entre 0,67 et 0,734.

Dans un autre mode de réalisation, le procédé selon l'invention permet d'obtenir une vanilline naturelle de pureté moindre, par exemple de pureté de 96 à 98,9% et comprenant des impuretés conférant au flux de vanilline naturelle obtenu des propriétés organoleptiques différentes de celle de la vanilline naturelle très pure et proches de celles de la vanille gousse. Dans ce mode de réalisation, le flux final de vanilline naturelle peut comprendre jusqu'à 3% en poids d'impuretés. Parmi les impuretés on retrouve en majorité l'alcool vanillique, et les dimères et/ou trimères, notamment les diphényleméthanes, qui peuvent représenter jusqu'à 1% en poids du flux, et en minorité l'acide vanillique, le gaïacol, l'acide benzoïque et les composés organiques biosynthétisés, qui peuvent être présents chacun jusqu'à 500 ppm dans le flux. Ce mode de réalisation peut être obtenu en adaptant les paramètres de distillation, notamment le ratio du débit de distillation *(F14)* et du débit d'alimentation *(F2)* est alors de préférence compris entre 0,735 et 0,8.

L'invention concerne également un dispositif pour la mise en œuvre de ce procédé comprenant :
- un premier dispositif de séparation (1) des ultra-légers, notamment l'acétate d'éthyle, d'un flux *(F1)* entrant dans ledit premier dispositif, ce premier dispositif étant notamment un échangeur à film tombant ou un réacteur double enveloppe ;
- un moyen de connexion du premier dispositif à une première colonne de distillation (2) pour transférer le flux sortant du premier dispositif *(F2)* vers l'entrée ladite première colonne ;
- une première colonne de distillation (2) de séparation d'une part des légers et d'autre part de la vanilline naturelle et des lourds, ladite première colonne comprenant une entrée du flux *(F2)* provenant du premier dispositif et une sortie en tête de colonne comprenant les légers *(F12),* et une sortie en pied de colonne comprenant la vanilline naturelle et les lourds *(F13) ;*
- un moyen de connexion de la première colonne à une seconde colonne de distillation (3) pour transférer le flux sortant en pied de la première colonne vers l'entrée de ladite seconde colonne ;
- une seconde colonne de distillation (3) de séparation d'une part de la vanilline naturelle et d'autre part des lourds, ladite seconde colonne comprenant une entrée d'un flux *(F13)* provenant de la première colonne et une sortie en tête de colonne comprenant la vanilline naturelle *(F14),* et une sortie en pied de colonne comprenant les lourds *(F15).*

Le dispositif peut également contenir une pompe (4) permettant de transférer le flux issu du premier dispositif vers la première colonne de distillation.

Le dispositif peut également contenir un condenseur relié à l'une des sorties du premier dispositif et permettant de récupérer les ultra-légers et/ou un condenseur relié à la tête de colonne de la colonne (2) permettant de récupérer les légers et/ou un condenseur relié à la tête de colonne (3) permettant de récupérer la vanilline naturelle.

Les caractéristiques concernant le premier dispositif et les colonnes de distillation sont précisées ci-dessus.

### ■ Equeutage/étêtage

Dans un mode de réalisation particulier le procédé mené par distillation continue comprend les étapes suivantes :
a) dans une première étape, le flux *(F1)* est traité afin d'éliminer les ultra-légers (flux *(F11)*) qu'il contient et plus particulièrement l'acétate d'éthyle, le flux ainsi obtenu comprenant la vanilline naturelle, les lourds et les légers est appelé flux *(F2) ;*
b) dans une deuxième étape, le flux *(F2)* est alimenté dans une première colonne de distillation, permettant de récupérer en tête de distillation un flux *(F22)* comprenant les légers et la vanilline naturelle et éventuellement une faible concentration de lourds et en pied de distillation un flux *(F23)* comprenant les lourds, notamment l'alcool vanillique, et éventuellement une faible concentration de vanilline naturelle ;
c) le flux *(F22)* est alimenté de façon continue dans une seconde colonne de distillation permettant de récupérer en tête de distillation un flux *(F24)* comprenant les légers et éventuellement une faible concentration en vanilline naturelle et en pied de distillation, un flux *(F25)* comprenant la vanilline naturelle et éventuellement une faible concentration en légers et/ou en lourds.

Dans ce mode de réalisation, on entend par « faible concentration » une concentration de préférence d'au plus 10%, de préférence d'au plus 5%, par exemple d'au plus 3% en poids par rapport au poids total de la fraction.

Selon un mode de réalisation préféré du schéma équeutage/étêtage, une partie au moins du flux (F23) est mélangé au flux (F25) de manière à pouvoir faire varier la composition finale d'un flux de vanilline naturelle purifiée.

De façon préférée, le flux *(F22)* en tête de distillation à l'étape (b) comprend les légers, notamment l'acide benzoïque, le gaïacol ; la vanilline naturelle et éventuellement une faible concentration en lourds. En pied de distillation *(F23)* on retrouve les lourds, notamment l'alcool vanillique et éventuellement une faible concentration en vanilline naturelle.

L'homme du métier est parfaitement en mesure de fixer les paramètres de distillation tels que notamment le diamètre de la colonne, la hauteur de colonne et le garnissage. On rappellera simplement ce qui suit. La taille (notamment le diamètre) des colonnes de distillation dépend du flux circulant et de la pression interne. Leur dimensionnement se fera donc principalement suivant le débit de mélange à traiter. On précisera que la colonne peut être garnie indifféremment de plateaux ou de garnissage ordonné ou tissé, comme cela est parfaitement connu de l'homme du métier. L'installation étant déterminée, l'homme du métier ajuste les paramètres de fonctionnement de la colonne.

De préférence, la colonne de l'étape b) comprend de 10 à 30 plateaux théoriques, de préférence de 15 à 25 plateaux théoriques. La colonne de distillation de l'étape b) comprend notamment de 4 à 14 plateaux théoriques pour la section d'épuisement et de 6 à 16 plateaux théoriques pour la section de concentration.

De préférence la colonne de l'étape b) est équipée par exemple d'un garnissage ordonné tissé. Le point d'entrée du flux *(F2)* se situe entre le cinquième et le quinzième plateau théorique en partant du bas.

Dans la colonne de l'étape b), la pression de service est de préférence comprise entre 2 et 10 mbar, de préférence entre 3 et 6 mbar, par exemple 4 mbar. La perte de charge est de préférence de 1 à 10 mbar, de préférence de 1 à 8 mbar, par exemple elle est de 4 mbar. Lors de l'étape b) le taux de reflux est compris entre 2 et 10, de préférence entre 2,5 et 6, il est par exemple de 3.

Le ratio du débit de distillation (débit du flux *(F22)*) sur le débit d'alimentation (débit du flux *(F2)*) est de préférence de 0,5 à 0,95.
Le débit de reflux est de préférence de 1,4 à 3 fois le débit d'alimentation, par exemple il est de 2 fois le débit d'alimentation.
De préférence au cours de l'étape b), la température de pied de colonne est comprise entre 160°C et 200°C, notamment entre 170°C et 190°C. De préférence au cours de l'étape b) la température en tête de colonne est comprise entre 100°C et 160°C, de préférence entre 110°C et 140°C.

Le temps de séjour de la vanilline en pied de colonne de l'étape b) est compris entre 15 minutes et 15 heures, de préférence entre 30 minutes et 15 heures et de manière encore plus préférée entre 7 et 15 heures. Afin de réduire ce temps de séjour entre 15 minutes et 8 heures et limiter la dégradation de la vanilline naturelle, qui est thermosensible, il peut être intéressant d'ajouter en pied de colonne un corps mort. Les corps morts sont bien connus de l'homme du métier, ils peuvent notamment consister en des billes de verre.

Pour effectuer la distillation de l'étape b) l'apport des calories en pied de colonne peut être fait notamment par un bouilleur à film tombant ou à film raclé, par un échangeur tubes-calandre chauffé à la vapeur ou par un fluide caloporteur, par l'intermédiaire de serpentins de chauffage alimentés à la vapeur ou par un fluide caloporteur ou par tout autre dispositif équivalent, de préférence par un fluide caloporteur. De préférence, l'énergie pour la distillation est apportée par un tubes-et-calandre à circulation forcée ou un échangeur raclé.

Le flux *(F22)* en tête de colonne en fin d'étape b) est envoyé vers la colonne de distillation de l'étape c) appelée également colonne d'étêtage. Ce flux peut être envoyé sous forme liquide, les vapeurs provenant de la colonne de l'étape b) étant totalement condensées à l'aide d'un condenseur, ou sous forme vapeur, les vapeurs provenant de la colonne de l'étape b) étant partiellement condensées à l'aide d'un condenseur partiel. De préférence, le flux *(F22)* est envoyé vers la colonne de distillation de l'étape c) en phase vapeur. Cela permet de façon avantageuse une économie directe de vapeur vive pour la colonne de distillation de l'étape c). Ainsi, la pression de service de la colonne de distillation de l'étape b) doit être supérieure à celle de la colonne de distillation de l'étape c) et un condenseur partiel est placé entre la colonne de distillation de l'étape b) et celle de l'étape c). Le condenseur partiel peut être tout type de condenseur connu de l'homme du métier, ce peut être par exemple un condenseur de type tubes et calandre.

De façon préférée, le flux *(F24)* en tête de distillation à l'étape c) comprend les légers, notamment l'acide benzoïque et le gaïacol, et éventuellement de la vanilline naturelle en faible concentration.

De préférence, la colonne de distillation de l'étape c) comprend de 20 à 35 plateaux théoriques, de préférence de 23 à 30 plateaux théoriques. La colonne de distillation de l'étape b) comprend notamment de 8 à 15 plateaux théoriques pour la section de concentration et de 12 à 20 plateaux théoriques pour la section d'épuisement, de préférence de 10 à 13 plateaux théoriques pour la section de concentration et de 13 à 17 plateaux théoriques pour la section d'épuisement. La colonne de distillation c) peut être une colonne de distillation équipée d'un garnissage ordonné sur une hauteur de 2x2600 mm à 2x3000 mm (2600 à 3000 mm pour chacune des sections d'épuisement et de concentration). Le garnissage de la colonne est de préférence un garnissage tissé du fait des très basses pressions de mise en œuvre.
Dans la colonne de l'étape c), la pression de service est de préférence comprise entre 2 et 10 mbar, de préférence entre 3 et 6 mbar, par exemple 4 mbar. La perte de charge est de préférence de 1 à 10 mbar, de préférence de 1 à 8 mbar, par exemple elle est de 4 mbar. Lors de l'étape c) le taux de reflux est compris entre 10 et 30, de préférence entre 15 et 25, il est par exemple de 20.

Le point d'alimentation du flux *(F22)* est choisi de façon connue par l'homme du métier, il est notamment choisi de façon à éviter les rétro-mélanges. De préférence, le point d'alimentation se trouve en haut de la section d'épuisement.

Le ratio du débit de distillation (débit du flux *(F24)*) sur le débit d'alimentation (débit du flux *(F2)*) est de préférence de 0,05 à 0,5.
Le débit de reflux est de préférence de 1,4 à 3 fois le débit d'alimentation, par exemple il est de 2 fois le débit d'alimentation.

De préférence au cours de l'étape c), la température de pied de colonne est comprise entre 100°C et 160°C, notamment entre 120°C et 150°C. De préférence au cours de l'étape c) la température en tête de colonne est comprise entre 80°C et 120°C, de préférence entre 90°C et 110°C.

Le temps de séjour de la vanilline en pied de la colonne de l'étape c) est compris entre 30 minutes et 5 heures, il est par exemple de 3 heures.

La distillation est démarrée en ajustant le débit de flux *(F22)* pour conserver une température sensible de 100 à 160 °C. L'écart de température entre la tête (température vapeurs) et le pied (température du liquide) est compris entre 35 et 45°C.

Pour effectuer la distillation de l'étape c) l'apport des calories en pied de colonne peut être fait notamment par un bouilleur à film tombant ou à film raclé, par un échangeur tubes-calandre chauffé à la vapeur ou par un fluide caloporteur, par l'intermédiaire de serpentins de chauffage alimentés à la vapeur ou par un fluide caloporteur ou par tout autre dispositif équivalent, de préférence par un fluide caloporteur. De préférence l'énergie pour la distillation est apportée par un bouilleur de type à film tombant, un tubes-et-calandre à circulation forcée ou un tubes-et-calandre à thermosiphon.

De préférence :
- le débit du flux *(F1)* est de 150 à 220 kg/h ;
- le débit du flux *(F11)* est de 100 à 180 kg/h ;
- le débit du flux *(F2)* est de 40 à 55 kg/h ;
- le débit du flux *(F22)* est de 33 à 52 kg/h ;
- le débit du flux *(F23)* est de 7 à 17 kg/h ;
- le débit du flux *(F25)* est de 20 à 60 kg/h.

Dans un mode de réalisation, le procédé selon l'invention permet d'obtenir une vanilline naturelle très pure, notamment d'une pureté supérieure ou égale à 99% et contenant de préférence moins de 1000 ppm d'alcool vanillique. Ce mode de réalisation peut être obtenu en adaptant les paramètres de distillation, notamment le ratio du débit de distillation *(F24)* et du débit d'alimentation *(F2)* est alors de préférence compris entre 0,06 à 0,083.

Dans un autre mode de réalisation, le procédé selon l'invention permet d'obtenir une vanilline naturelle de pureté moindre, par exemple de pureté de 96 à 98,9% et comprenant des impuretés conférant au flux de vanilline naturelle obtenu des propriétés organoleptiques différentes de celle de la vanilline naturelle très pure et proches de celles de la vanille gousse. Dans ce mode de réalisation, le flux final de vanilline naturelle peut comprendre jusqu'à 3% en poids d'impuretés. Parmi les impuretés on retrouve en majorité l'alcool vanillique, et les dimères et/ou trimères, notamment les diphényleméthanes, qui peuvent représenter jusqu'à 1% en poids du flux, et en minorité l'acide vanillique, le gaïacol, l'acide benzoïque et les composés organiques biosynthétisés, qui peuvent être présents chacun jusqu'à 500 ppm dans le flux. Ce mode de réalisation peut être obtenu en adaptant les paramètres de distillation, notamment le ratio du débit de distillation *(F24)* et du débit d'alimentation *(F2)* est alors de préférence compris entre 0,084 à 0,1.

L'invention concerne également un dispositif pour la mise en œuvre de ce procédé comprenant :
- un premier dispositif (1) de séparation des ultra-légers, notamment l'acétate d'éthyle, d'un flux *(F1)* entrant dans ledit premier dispositif, ce premier dispositif étant notamment un échangeur à film tombant ou un réacteur double enveloppe ;
- un moyen de connexion du premier dispositif à une première colonne de distillation (5) pour transférer le flux *(F2)* sortant du premier dispositif vers l'entrée de ladite première colonne ;
- une première colonne de distillation (5) de séparation d'une part des légers et de la vanilline, et d'autre part des lourds ladite première colonne comprenant une entrée du flux *(F2)* provenant du premier dispositif et une sortie en tête de colonne comprenant les légers et la vanilline naturelle *(F22),* et une sortie en pied de colonne comprenant les lourds *(F23) ;*

- un moyen de connexion de la première colonne à une seconde colonne de distillation (6) pour transférer le flux *(F22)* sortant en tête de la première colonne vers l'entrée de la seconde colonne ;
- une seconde colonne de distillation (6) de séparation d'une part de la vanilline naturelle et d'autre part des légers, ladite seconde colonne comprenant une entrée d'un flux *(F22)* provenant de la première colonne et une sortie en tête de colonne comprenant les légers *(F24),* et une sortie en pied de colonne comprenant la vanilline naturelle *(F25).*

Le dispositif peut également contenir une pompe (4) permettant de transférer le flux issu du premier dispositif vers la première colonne de distillation.

Le dispositif peut également contenir un condenseur relié à l'une des sorties du premier dispositif et permettant de récupérer les ultra-légers et/ou un condenseur relié à la tête de colonne de la colonne (6) permettant de récupérer les légers.

Dans le cas où le flux d'entrée de la seconde colonne de distillation est introduit en phase vapeur, le dispositif comprend également un condenseur partiel reliant la tête de colonne de la première colonne (5) et le point d'alimentation de la deuxième colonne (6).
Dans le cas où le flux d'entrée de la seconde colonne de distillation est introduit en phase liquide, le dispositif comprend également un condenseur reliant la tête de colonne de la première colonne (5) et le point d'alimentation de la deuxième colonne (6).

Les caractéristiques concernant le premier dispositif, le condenseur et les colonnes de distillation sont précisées ci-dessus.

Le mode de réalisation comprenant la mise en œuvre d'un condenseur partiel permet d'importantes économies d'énergie notamment dans le cadre de production importante.

### Distillation continue par colonne à cloison (ou DWC)

Dans un mode de réalisation, le procédé selon l'invention est mené par distillation continue à l'aide d'une colonne à cloison.

Le procédé mené par distillation continue par colonne à cloison comprend les étapes suivantes :
a) dans une première étape, le flux *(F1)* est traité afin d'éliminer les ultra-légers (flux *(F11))* qu'il contient et plus particulièrement l'acétate d'éthyle, le flux résultant comprenant la vanilline naturelle, les légers et les lourds ainsi obtenu est appelé flux *(F2) ;*
b) dans une seconde étape, le flux *(F2)* est alimenté dans une colonne à cloison qui permet de récupérer en tête de colonne un flux *(F32)* comprenant les légers et éventuellement une faible concentration de vanilline naturelle, en pied de colonne un flux *(F34)* comprenant les lourds, et notamment l'alcool vanillique, et éventuellement une faible concentration en vanilline naturelle, la vanilline étant récupérée sous la forme d'un flux *(F33)* par soutirage latéral, ce flux pouvant éventuellement comprendre une faible concentration de légers et/ou de lourds.

Dans ce mode de réalisation, on entend par « faible concentration » une concentration de préférence d'au plus 10%, de préférence d'au plus 5%, par exemple d'au plus 3% en poids par rapport au poids total de la fraction.

Le soutirage latéral peut être effectué en phase liquide et/ou en phase vapeur. Ceci peut être effectué par toute méthode connue de l'homme du métier. Par exemple :
- en phase liquide, un tampon de garnissage peut être remplacé par un bac de récupération à débordement ;
- en phase vapeur, par une liaison avec un condenseur latéral dont la pression d'évent est en équilibre avec la pression de tête ;
De façon avantageuse, le soutirage latéral en phase vapeur évite d'entrainer les lourds dans le flux de vanilline naturelle obtenue.
De façon avantageuse, le soutirage latéral en phase liquide permet une économie énergétique puisque la soustraction d'enthalpie est bien moins importante qu'un soutirage en phase vapeur.
Le soutirage en phase vapeur permet de façon avantageuse d'augmenter la pureté de la vanilline obtenue. Le soutirage en phase liquide permet quant à lui, de façon avantageuse, de conserver des impuretés dans le flux de vanilline naturelle finale permettant ainsi l'obtention de propriétés organoleptiques différentes.

De façon avantageuse, le procédé selon l'invention peut comprendre deux soutirages latéraux simultanés, un soutirage latéral en liquide et un soutirage latéral en phase vapeur. Cela permet en une seule étape de distillation de recueillir des flux de vanilline naturelle de pureté différente et donc possédant des propriétés organoleptiques différentes.

Dans le cadre de la présente invention on entend par colonne à cloison une colonne de distillation comprenant un segment de séparation interne vertical séparant la colonne en deux demi-colonne de géométrie et de volume indépendant l'un de l'autre, une demi-colonne primaire dans laquelle a lieu l'alimentation du flux *(F2)* et une demi-colonne secondaire dans laquelle a lieu le soutirage du flux *(F34),* les deux demi-colonnes communiquent entre-elles, le segment de séparation n'allant pas d'une extrémité à l'autre de la colonne.

De préférence, la colonne à cloison est telle que la demi-colonne primaire comprend entre 18 et 30 étages théoriques, de préférence entre 25 et 30 étages théoriques, par exemple 27 étages théoriques ; et la demi-colonne secondaire comprend entre 18 et 25 étages théoriques, de préférence entre 20 et 25 étages théoriques, par exemple 23 étages théoriques. De préférence, la demi-colonne primaire comprend de 10 à 20 étages théoriques pour la section d'épuisement et de 8 à 10 étages théoriques pour la section de concentration. De préférence, la demi-colonne secondaire comprend de 10 à 20 étages théoriques au dessus du point de soutirage latéral en phase vapeur et 10 à 15 étages théoriques en dessous du point de soutirage en phase vapeur. Le soutirage phase liquide se trouve 1 à 4 plateaux en-dessous du soutirage phase vapeur.
De préférence, la pression de service de la colonne à cloison est comprise entre 2 et 10 mbar, de préférence entre 3 et 6 mbar, par exemple 4 mbar. La perte de charge doit de préférence être identique dans chacune des deux demi-colonnes formant la colonne à cloison, pour cela le diamètre de chaque demi-colonne est adapté. La perte de charge dans chaque demi-colonne est de 0,5 à 2,5 mbar.
Le taux de reflux dans chaque demi-colonne de la colonne à cloison, identique ou différent, est de préférence compris entre 2 et 10, de préférence entre 2,7 et 6

De préférence l'alimentation du flux *(F2)* a lieu entre le dixième et le vingtième étage théorique de la demi-colonne primaire, les plateaux étant comptés de bas en haut. La vanilline est soutirée en latéral de la colonne à cloison, de préférence entre le quatrième et le quinzième étage théorique de la demi-colonne secondaire, les plateaux étant comptés de bas en haut.
De préférence, le segment de séparation comprend un orifice permettant le passage d'un flux enrichi en vanilline de la demi-colonne primaire vers la demi-colonne secondaire. L'orifice se trouve de préférence au moins deux plateaux théoriques au dessus de l'alimentation par rapport à la demi-colonne primaire, de préférence il est entre 2 et 5 plateaux au-dessus de l'alimentation, les plateaux étant comptés par rapport à la demi-colonne primaire. La taille de l'orifice est déterminée pour laisser passer un flux enrichi en vanilline et pauvre en légers, la taille de l'orifice peut être déterminée par l'homme du métier. Elle est de préférence de 5 à 20 mm, par exemple de 8 à 15 mm, elle est notamment d'environ 10 mm, de large sur la largeur du segment de séparation.

Le temps de séjour en pied de colonne est de préférence de 30 minutes à 13 heures, par exemple 8 heure. Afin de réduire ce temps de séjour entre 1 et 7 heures et limiter la dégradation de la vanilline naturelle, qui est thermosensible, il peut être intéressant d'ajouter en pied de colonne un corps mort. Les corps morts sont bien connus de l'homme du métier, ils peuvent notamment consister en des billes de verre.

Pour effectuer la distillation l'apport des calories en pied de colonne peut être fait notamment par un bouilleur à film tombant ou à film raclé, par un échangeur tubes-calandre chauffé à la vapeur ou par un fluide caloporteur, par l'intermédiaire de serpentins de chauffage alimentés à la vapeur ou par un fluide caloporteur ou par tout autre dispositif équivalent, de préférence par un fluide caloporteur.

De préférence, le ratio débit soutirage latéral total (vapeur et/ou liquide) par rapport au débit de flux *(F2)* est de 0,5 à 0.95.

De préférence :
- le débit du flux *(F1)* est de 150 à 220 kg/h ;
- le débit du flux *(F2)* est de 40 à 70 kg/h ;
- le débit du flux *(F32)* est de 3 à 7 kg/h ;
- le débit du flux *(F33)* en phase vapeur est de 20 à 60 kg/h.

Lorsque la colonne comporte un double soutirage vapeur et liquide, le débit du flux vapeur est de 35 à 45 kg/h et le débit du flux liquide est de 1,0 à 2,5 kg/h.

Dans un mode de réalisation, le procédé selon l'invention permet d'obtenir une vanilline naturelle très pure, notamment d'une pureté supérieure ou égale à 99% et contenant de préférence moins de 1000 ppm d'alcool vanillique. Ce mode de réalisation est obtenu en soutirant la vanilline naturelle sous forme vapeur. Le ratio du débit de distillation *(F33)* et du débit d'alimentation *(F2)* est alors de préférence compris entre 0,5 à 0,734.

Dans un autre mode de réalisation, le procédé selon l'invention permet d'obtenir une vanilline naturelle de pureté moindre, par exemple de pureté de 96 à 98,9% et comprenant des impuretés conférant au flux de vanilline naturelle obtenu des propriétés organoleptiques différentes de celle de la vanilline naturelle très pure et proches de celles de la vanille gousse. Dans ce mode de réalisation, le flux final de vanilline naturelle peut comprendre jusqu'à 3% en poids d'impuretés. Parmi les impuretés on retrouve en majorité l'alcool vanillique, et les dimères et/ou trimères, notamment les diphényleméthanes, qui peuvent représenter jusqu'à 1% en poids du flux, et en minorité l'acide vanillique, le gaïacol, l'acide benzoïque et les composés organiques biosynthétisés, qui peuvent être présents chacun jusqu'à 500 ppm dans le flux. Le ratio du débit de distillation *(F33)* et du débit d'alimentation *(F2)* est alors de préférence compris entre 0,736 et 0,76.

Le procédé de l'invention permet également d'obtenir par une seule étape de distillation la vanilline très pure, de pureté supérieure ou égale à 99%, et la vanilline présentant une pureté de 96 à 98,9% en prévoyant deux soutirages latéraux, un soutirage en phase vapeur permettant d'obtenir la vanilline de pureté supérieure à 99%, et un soutirage en phase liquide permettant de récupérer la vanilline de pureté 96 à 98,9%.

La présente invention concerne également un dispositif pour la mise en œuvre de ce procédé comprenant :
- un premier dispositif (1) de séparation des ultra-légers, notamment l'acétate d'éthyle, d'un flux *(F1)* entrant dans ledit premier dispositif, ce premier dispositif étant notamment un échangeur à film tombant ou un réacteur double enveloppe ;
- un moyen de connexion du premier dispositif à une colonne de distillation à cloison (7) pour transférer le flux sortant du premier dispositif *(F2)* vers l'entrée de ladite colonne à cloison ;
- une colonne de distillation à cloison (7) de séparation des légers, des lourds et de la vanilline naturelle, ladite colonne à cloison comprenant une entrée du flux *(F2)* provenant du premier dispositif et une sortie en tête de colonne comprenant des légers *(F32),* une sortie en pied de colonne comprenant les lourds *(F34),* et un ou deux dispositif de soutirage latéral de la vanilline naturelle *(F33).*

Le dispositif contient également une pompe (4) permettant de transférer le flux issu du premier dispositif vers la colonne à cloison.

La colonne à cloison comprend un segment interne (8) séparant la colonne en deux demi-colonnes (71) et (72). Le segment comprend un orifice (9) tel que décrit ci-dessus.

Les caractéristiques concernant le premier dispositif et la colonne à cloison sont précisées ci-dessus.

Le procédé par colonne à cloison est la variante préférée de l'invention.
De façon générale dans les procédés de l'invention l'étape a) peut être mise en œuvre par exemple par l'intermédiaire d'un échangeur à film tombant ou d'un réacteur coquillé (ou réacteur à double enveloppe), de préférence un échangeur à film tombant.
On entend par évaporateur à film tombant (dénommé également évaporateur à film ruisselant), un appareil constitué par une chambre généralement cylindrique comprenant un faisceau de tubes verticaux ; lesdits tubes étant chauffés extérieurement par circulation de vapeur dans ladite chambre. Le flux *(F1)* est alimenté par la partie supérieure et tombe sur un système de distribution par exemple avec déversoir permettant une bonne répartition du liquide au sommet des tubes pour constituer un film liquide d'une fine épaisseur, généralement inférieure à 1 mm, de préférence compris entre 0,3 et 0,5 mm. Au cours de cette étape préalable, la pression de service est de préférence la pression atmosphérique.
La surface de l'échangeur est de préférence de 0,3 à 0,6 m² pour un débit d'alimentation d'environ 200 kg/h. Pour un débit différent l'homme du métier est capable de part ses connaissances générales de déterminer la surface de l'échangeur.
Les ultra-légers, notamment l'acétate d'éthyle peuvent être récupérés à l'aide d'un condenseur pour une utilisation ultérieure.
Au pied de l'évaporateur à film tombant une pompe de reprise permet d'envoyer le flux liquide *(F2)* récupéré en sortie vers la colonne de distillation de l'étape b) appelée également colonne à cloison. Le flux *(F2)* correspond au flux *(F1)* substantiellement ou totalement exempt d'ultra-légers. Le flux *(F2)* peut éventuellement contenir des traces d'ultra-légers qui seront piégés dans le piège à froid de la colonne de l'étape b).

Dans un deuxième aspect particulier, l'invention a pour objet un procédé de purification de vanilline naturelle ayant une pureté allant de 95 à 99% en poids pour obtenir de la vanilline naturelle faiblement colorée, comprenant les étapes suivantes :
(a) faire fondre la vanilline naturelle ;
(b) soumettre la vanilline fondue issue de l'étape (a) à une étape d'évaporation sous vide ;
(c) récupérer un condensat de vanilline naturelle purifiée. Dans ce deuxième aspect particulier l'on emploie une vanilline naturelle ayant une pureté allant de 95 à 99% en poids, c'est-à-dire comprenant de 95 à 99% en poids de vanilline naturelle. Elle peut comprendre en outre, typiquement, de 1 à 5% en poids d'impuretés comprenant des oligomères phénoliques. Parmi les impuretés comprenant des oligomères phénoliques, on peut citer en particulier les dimères et les trimères de la vanilline.

La vanilline naturelle employée dans le procédé selon l'invention a donc généralement été préalablement purifiée, afin d'atteindre le degré de pureté de 95 à 99% en poids.

Une telle purification peut avoir été effectuée par toute méthode connue permettant d'atteindre, à partir d'une vanilline naturelle brute, c'est-à-dire directement issue de la biosynthèse, un tel degré de pureté. De préférence, la vanilline a été préalablement purifiée par distillation, en particulier telle que décrite ci-dessus dans le cadre du premier aspect du procédé selon l'invention. Le cas échéant, une extraction par solvant organique éventuellement suivie d'une précipitation ou cristallisation de la vanilline naturelle peut être mise en œuvre pour effectuer une telle purification.

De manière préférée, le deuxième aspect du procédé selon l'invention emploie une vanilline naturelle obtenue par distillation de vanilline naturelle brute issue d'un procédé de biosynthèse.

De préférence, cette étape de purification préalable aura permis d'éliminer entre autres les composés dont la volatilité est plus élevée que celle de la vanilline naturelle dans les conditions de pression et de température considérées. Ces composés, qui sont issus des procédés de préparation de la vanilline naturelle, peuvent être entre autres l'acide benzoïque, l'alcool vanillique, le gaïacol et un solvant, de préférence un solvant alimentaire tel que l'acétate d'éthyle.
Par volatilité, on désigné de manière connue en soi la capacité d'une substance à se vaporiser.
Dans le deuxième aspect du procédé selon l'invention, la vanilline naturelle brute directement issue de la biosynthèse comprend typiquement :
- de la vanilline naturelle en une proportion pouvant aller de 5 à 35% en poids, notamment de 10 à 30 % en poids ;
- de l'alcool vanillique en une proportion pouvant aller de 0,05 à 10 % en poids, de préférence de 0,1 à 5 % en poids;
- de l'acide vanillique en une proportion pouvant aller de 0 à 2 % en poids, de préférence de 0,01 à 0,6 % en poids ;
- de l'acide férulique en une proportion pouvant aller de 0 à 2 % en poids, de préférence de 0,01 à 0,9 % en poids ;
- du gaïacol en une proportion pouvant aller de 0 à 2 % en poids, de préférence de 0,01 à 0.6 % en poids ;
- des dimères et des trimères en une proportion pouvant aller de 0 à 5% en poids, de préférence de 0,05 à 5 % en poids, de préférence encore de 0,1 à 5% en poids ; les dimères et trimères étant des composés présentant un squelette ayant respectivement deux ou trois groupements phényles, les dimères étant avantageusement choisis parmi les diphényleméthanes ;
- divers composés organiques également biosynthétisés (comme par exemple le 4-méthyle gaïacol, le 4-éthyle gaïacol et/ou le 4-vinyle gaïacol) en une proportion pouvant aller de 0,0 à 5% en poids, de préférence de 0,01 à 5 % en poids, de préférence encore de 0,05 à 1% en poids ;
- de l'acide benzoïque en une proportion pouvant aller de 0 à 10% en poids, de préférence de 0,01 à 10 % en poids, de préférence encore de 0,05 à 5% en poids ;
- de l'eau en une proportion pouvant aller de 0 à 5% en poids, de préférence de 0,01 à 5 % en poids, de préférence encore de 0,05 à 3 % en poids ;
- le complément à 100% en poids d'un solvant alimentaire, en particulier l'acétate d'éthyle.

Dans le deuxième aspect du procédé selon l'invention, de préférence, le procédé de biosynthèse dont est issue la vanille naturelle mise en œuvre dans l'invention confère à cette vanilline naturelle des propriétés organoleptiques proches de celles de la vanille gousse.

De manière particulièrement préférée, le deuxième aspect du procédé selon l'invention emploie une vanilline naturelle présentant une pureté allant de 96 à 98,9% en poids. Selon une autre caractéristique de l'invention, le deuxième aspect du procédé selon l'invention comprend une étape supplémentaire (d) consistant en la récupération d'un résidu comprenant des oligomères phénoliques, tels que ceux décrits ci-avant.

Dans un mode de réalisation préféré du deuxième aspect du procédé selon l'invention, la vanilline naturelle est fondue lors de l'étape (a) à une température allant de 70 à 110°C et à pression atmosphérique, plus préférentiellement à une température allant de 75 à 100°C et à pression atmosphérique.

Dans le deuxième aspect du procédé selon l'invention, l'étape (a) est avantageusement effectuée sous atmosphère de gaz inerte, c'est-à-dire présentant de préférence une teneur en oxygène inférieure ou égale à 1% en volume. Des gaz inertes appropriés sont l'azote, l'argon et leurs mélanges.

Selon une autre caractéristique du deuxième aspect du procédé selon l'invention, l'étape (b) d'évaporation est avantageusement effectuée sous une pression allant de 1 à 10 mbar et à une température allant de 110 à 160°C.
De préférence encore, l'étape (b) d'évaporation est effectuée sous une pression allant de 1 à 6 mbar. De manière également préférée, l'étape (b) d'évaporation est effectuée à une température allant de 115 à 135°C.

Selon un mode de réalisation particulièrement avantageux du deuxième aspect du procédé selon l'invention, l'étape (b) de l'invention est effectuée dans un évaporateur. Celui-ci peut être plus particulièrement choisi parmi un évaporateur à film raclé ou un évaporateur à film tombant. De manière particulièrement préférée, l'étape (b) est effectuée dans un évaporateur à film raclé.

Des évaporateurs utilisables à cet effet sont ainsi des évaporateurs à couche mince comme des évaporateurs à film tombant ou raclé. De tels évaporateurs sont commercialement disponibles, par auprès des sociétés BUSS AG, GEA CANZLER ou KUHNI, et sont bien connus de l'homme du métier. Dans une variante particulièrement préférée du deuxième aspect du procédé selon l'invention, un évaporateur à film raclé court-trajet, dont le principe repose sur un faible temps de passage du produit à évaporer, est mis en œuvre. L'évaporateur à film raclé s'est avéré particulièrement bien adapté à une purification par évaporation de la vanilline naturelle, et permet d'éviter toute dégradation de ce produit thermosensible.

La vanilline naturelle fondue issue de l'étape (a) est alors introduite au sein d'un tel évaporateur. Une couche mince de phase liquide, comprenant la vanilline naturelle, est évaporée sur une surface chauffée et la phase liquide comprenant des oligomères phénoliques est raclée par un moyen mécanique de type palettes ou rouleaux. Cette dernière peut être ensuite récupérée dans un réceptacle dédié.

L'évaporateur permet de récupérer un effluent gazeux contenant la vanilline évaporée, lequel est ensuite condensé de manière à récupérer la vanilline purifiée.

Avantageusement, le temps de passage de la vanilline naturelle dans l'évaporateur est inférieur ou égal à 15 minutes, de préférence inférieur ou égal à 10 minutes, de préférence encore inférieur ou égal à 5 minutes.

Selon une caractéristique du deuxième aspect du procédé selon l'invention, le condensat de vanilline naturelle récupéré à l'étape (c) présente une pureté supérieure ou égale à 99% en poids, de préférence supérieure ou égale à 99,5% en poids.

Avantageusement, le condensat de vanilline naturelle purifiée obtenu par le procédé selon l'invention, en particulier le premier ou deuxième aspect particulier est ensuite directement mis en forme par solidification directe, de préférence par écaillage, pastillage ou prilling (agglomération).

Une des techniques de mise en forme préférées du condensat de vanilline naturelle purifiée est la technique d'écaillage sur cylindre ou sur bande.

Dans cette technique, le condensat de vanilline naturelle purifiée est mis en contact avec un cylindre ou une bande métallique à une température de 50°C, puis en raclant avec un couteau le film solide obtenu sur le cylindre, le condensat est récupéré sous forme d'écailles.

Une autre technique préférée est le pastillage qui consiste en un procédé d'agglomération de particules solides par solidification sur une bande de pastillage refroidie à 10°C. Ainsi, le condensat de vanilline naturelle purifiée se présente sous la forme d'un solide amorphe sec.

Le prilling est également une technique de mise en forme préférée. Il s'agit d'une technique bien connue de l'homme du métier consistant à pulvériser le condensat de vanilline dans un flux d'air ou d'azote froid pour obtenir des perles solides ou agglomérats (prills).

Le condensat de vanilline naturelle purifiée peut également être affiné par broyage ou tamisage. L'opération de broyage, notamment, peut être effectuée dans un appareillage classique tel qu'un broyeur à palettes, un broyeur à broches ou un granulateur.

Le procédé selon l'invention présente l'avantage de permettre l'obtention d'une vanilline d'une très grande pureté (supérieure à 99% en poids), et très faiblement colorée.

Pour caractériser la coloration de la vanilline obtenue, l'on peut procéder par mise en solution de celle-ci dans l'éthanol, à une concentration de 10% en poids.

Ainsi, le condensat de vanilline naturelle en solution éthanolique à 10% en poids est de couleur inférieure ou égale à 200 Hazen, de préférence inférieure ou égale à 100 Hazen. La couleur de la solution de vanilline peut être en particulier mesurée conformément à la norme ISO 6271 ou la norme ASTM D1209. On peut employer à cet effet un colorimètre Konica-Minolta CM-5.

L'invention a également pour objet la vanilline susceptible d'être obtenue par le procédé selon l'invention. Celle-ci se présente sous la forme d'un solide amorphe dont la couleur, en solution éthanolique à 10% en poids, est inférieure ou égale à 200 Hazen, et de préférence inférieure ou égale à 100 Hazen.
La figure 1 représente un schéma de distillation continue étêtage/équeutage d'un flux de vanilline naturelle *(F1).*
La figure 2 représente un schéma de distillation continue équeutage/étêtage d'un flux de vanilline naturelle *(F1).*
La figure 3 représente un schéma de distillation continue dans une colonne à cloison d'un flux de vanilline naturelle *(F1).*
La présente invention est illustrée de manière non-limitative par les exemples suivants.

### Exemples

Les exemples suivants décrivent la distillation d'un flux de vanilline *(F1)* comprenant 20% en poids de vanilline naturelle ; 4% en poids de lourds (acide vanillique, alcool vanillique, acide férulique, 4-méthyle gaïacol, 4 éthyle gaïacol, dimères et trimères, benzoate de sodium); 0,5% en poids d'eau ; 2,5% en poids de légers (acide benzoïque et gaïacol) ; 73% en poids d'ultra-légers (acétate d'éthyle).

### Exemple 1 : Distillation du flux (F1) par distillation discontinue

180 kg du flux *(F1)* sont alimentés dans le bouilleur d'une colonne de distillation de 700 mm de diamètre équipé de 10 plateaux théoriques, garnie d'un garnissage tissé de surface 450 m2/m3. Le fluide caloporteur est fixé au début à 110°C et le frigoporteur est une huile Gilotherm à 20°C. La colonne est mise au reflux total pendant 3 heures. On récupère ainsi une première fraction comprenant l'acétate d'éthyle au débit de 15 kg/heure avec un reflux de 0,5 pendant 10 heures. L'étape d'élimination de l'acétate est terminée lorsque la pression de service est de 4 mbar pour un fluide caloporteur de 160°C.

On récupère alors une seconde fraction comprenant les légers et 10% en poids de vanilline à un débit de 2,4 kg/heure avec un taux de reflux de 100 pendant une durée de 4 heures.

La colonne est remise en reflux total pendant 1 heure lorsque la température de tête de colonne atteint 139°C, le caloporteur est pour cela réglé à 165°C.

On récupère ensuite une fraction de vanilline naturelle comprenant la vanilline naturelle ayant une pureté de 98.2% et au débit de 6,1 kg/heure avec un reflux de 30 pendant une durée de 10 heures. La pression de service est de 4 mbar. Après 10h le débit de distillat est réduit à 3 kg/heure et le taux de reflux est fixé à 100. La durée de cette opération est de 0,5 heure. La distillation est arrêtée lorsque la température de tête atteint 143°C, la charge restante dans le culot est d'environ 9,6 kg et comprend les lourds. Le rendement de la purification est égal à 93%.

### Exemple 2 : Distillation du flux (F1) par distillation continue étêtage/équeutage

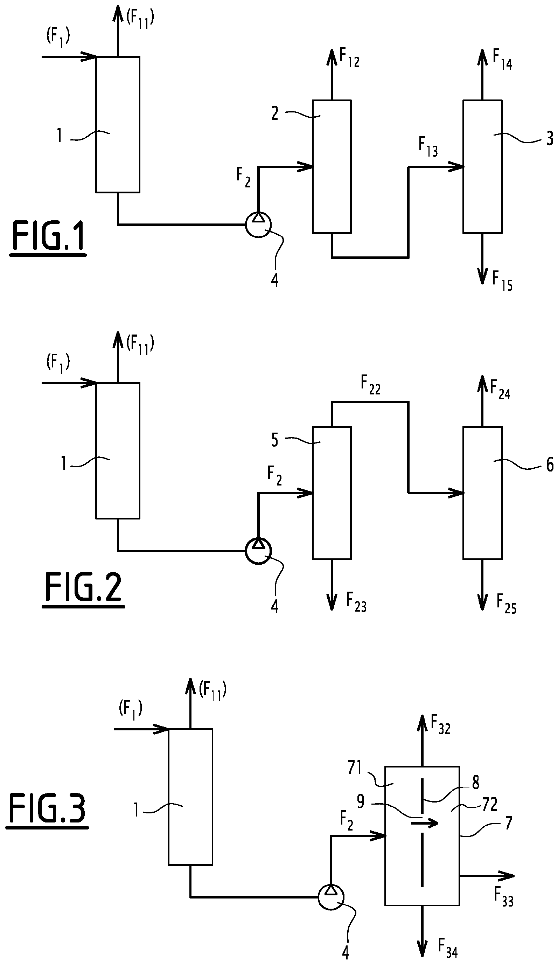

L'exemple 2 fait référence à la figure 1.
Le flux *(F1)* est alimenté au débit de 200 kg/heure en tête d'un échangeur à film tombant (1). Cet échangeur permet d'éliminer l'acétate d'éthyle sous un flux *(F11)* au débit de 146 kg/heure. Le flux *(F11)* est envoyé vers un condenseur (non représenté) pour récupération de l'acétate d'éthyle.
Un flux *(F2)* est récupéré en sortie de l'évaporateur qui correspond au flux *(F1)* substantiellement exempt d'acétate d'éthyle. Les traces d'acétate d'éthyle présentes dans le flux *(F2)* sont récupérées dans le piège à froid d'une colonne de distillation (2) permettant ainsi de protéger la colonne (2). Le flux *(F2)* est envoyé vers une colonne de distillation (étêtage) (2) par l'intermédiaire d'une pompe de reprise (4), partiellement en recycle, au débit de 54 kg/heure. En tête de colonne (2) on récupère un flux *(F12)* au débit de 5 kg/heure comprenant l'acide benzoïque, le gaiacol et 3% en poids de vanilline. En pied de colonne (2) on récupère un flux *(F13)* comprenant les lourds et la vanilline naturelle restante qui est envoyé vers une colonne d'équeutage (3) au débit de 49 kg/heure. En tête de colonne (3) on récupère un flux *(F14)* de vanilline naturelle comprenant 3 % en poids de lourds essentiellement constitués d'alcool vanillique et de dimères dont le diphénylméthane, à un débit de 40 kg/heure. La pureté de la vanilline est égale à 97%. Le rendement du procédé est égal à 97 %. En pied de colonne (3) on récupère un flux *(F15)* les lourds et 3% en poids de vanilline naturelle à un débit de 9kg/heure.

Les caractéristiques du dispositif sont les suivantes :
- Echangeur (1) : Surface = 0,5 m2.
- Colonne (2)

Equipée d'un garnissage ordonné (tissé à bas régime) sur une hauteur de 2x2600mm
24 plateaux théoriques divisés en 11 plateaux pour la section de concentration et 13 plateaux pour la section d'épuisement
Colonne de 4 mètres de hauteur
Pression de service : 4 mbar
Taux de reflux : 20

La colonne est dans un premier temps classée pendant 5 h avec un mélange vanilline (90 % poids), acide benzoïque (10 % poids). La distillation est ensuite démarrée en ajustant le débit de flux *(F12)* pour conserver une température sensible de 140°C. L'écart de température entre la tête (température vapeurs) et le pied (température du liquide) de 39.6°C sous 4 mbar. La colonne est en reflux total pour la phase de classement.

L'énergie pour la distillation est apportée par un bouilleur de type à film tombant, un tubes-et-calandre à circulation forcée ou un tubes-et-calandre à thermosiphon.
- Colonne 3

Equipée d'un garnissage ordonné tissé de surface environ 450 m2/m3, sur une hauteur de 2x4560 mm
Le point d'alimentation se trouve à la cote Z=1500 mm
18 plateaux théoriques
Diamètre de 600 mm
Pression de service : 4 mbar

La colonne est mise en reflux total à l'aide d'un pied de vanilline pure préalablement fondu à l'étuve
Taux de reflux : 3
Ratio [débit de distillat]/[débit d'alimentation] est de 0,808

L'écart de température entre la tête (température vapeurs) et le pied (température du liquide) est de 43°C sous 4 mbar.

L'énergie pour la distillation est apportée par un tubes-et-calandre à circulation forcée ou un échangeur raclé. La pression côté calandre est de 16 bar.

### Exemple 3 : Distillation du flux (F1) par distillation continue équeutage/étêtage

L'exemple 3 fait référence à la figure 2.
Le flux *(F1)* est alimenté au débit de 198,26 kg/heure en tête d'un échangeur à film tombant (1). Cet échangeur permet d'éliminer l'acétate d'éthyle sous un flux *(F11)* au débit de 147 kg/heure. Le flux *(F11)* est envoyé vers un condenseur (non représenté) pour récupération de l'acétate d'éthyle.
Un flux *(F2)* est récupéré en sortie de l'évaporateur qui correspond au flux *(F1)* substantiellement exempt d'acétate d'éthyle. Les traces d'acétate d'éthyle présentes dans le flux *(F2)* sont récupérées dans le piège à froid d'une colonne de distillation (5) permettant ainsi de protéger la colonne (5). Le flux *(F2)* est envoyé vers une colonne de distillation (équeutage) (5) par l'intermédiaire d'une pompe de reprise (4), partiellement en recycle, au débit de 51,26 kg/heure. En pied de colonne (5) on récupère un flux *(F23)* comprenant les lourds et 3% en poids de vanilline.

En tête de colonne (5) on récupère un flux *(F22)* au débit de 44 kg/heure comprenant les légers, la vanilline restante et 2,5% en poids de lourds qui est envoyé vers une colonne d'étêtage (6) au débit de 44kg/heure. En tête de colonne (6) on récupère un flux *(F24)* comprenant les légers et 3% en poids de vanilline. En pied de colonne (6) on récupère un flux *(F25)* de vanilline naturelle comprenant la vanilline et 2,5% en poids de lourds à un débit de 38,6 kg/h. La vanilline obtenue a une pureté de 99,9% et le rendement de purification est de 98,9%.

Les caractéristiques du dispositif sont les suivantes :
- Echangeur (1) : Surface = 0,5 m2.
- Colonne (5)

Equipée d'un garnissage ordonné tissé de surface environ 450 m2/m3, sur une hauteur de 2x4560 mm
Le point d'alimentation se trouve à la cote Z=1500 mm
18 plateaux théoriques
Diamètre de 600 mm
Pression de service : 4 mbar
Taux de reflux : 3
Ratio [débit de distillat]/[débit d'alimentation] est de 0.83
Le débit de reflux représente 2,49 fois le débit d'alimentation

L'écart de température entre la tête (température vapeurs) et le pied (température du liquide) est de 26°C sous 4 mbar

L'énergie pour la distillation est apportée par un tubes-et-calandre à circulation forcée ou un échangeur raclé. La pression côté calandre est de 12 bars.
- Colonne (6)

Equipée d'un garnissage ordonné (tissé à bas régime) sur une hauteur de 2x2600mm
24 plateaux théoriques divisés en 11 plateaux pour la section de concentration et 13 plateaux pour la section d'épuisement
Colonne de 4 mètres de hauteur
Pression de service : 4 mbar
Taux de reflux : 20

La colonne est dans un premier temps classée pendant 5 h avec un mélange vanilline (90 % poids), acide benzoïque (10 % poids). La distillation est ensuite démarrée en ajustant le débit de flux *(F24)* pour conserver une température sensible de 140°C. L'écart de température entre la tête (température vapeurs) et le pied (température du liquide) de 35°C sous 4 mbar. La colonne est en reflux total pour la phase de classement

L'énergie pour la distillation est apportée par un bouilleur de type à film tombant, un tubes-et-calandre à circulation forcée ou un tubes-et-calandre à thermosiphon. La pression côté calandre est de 10 bar.

Une alternative consiste à alimenter la colonne (6) en phase vapeur : on utilise pour cela une condensation partielle via un condenseur partiel. Cette alternative est une économie directe de vapeur vive pour la colonne (6). La colonne (5) fonctionne alors à 5 mbar ; la colonne (6) restant à 4 mbar. Dans ce cas, la température de tête affiche 139°C. Le condenseur partiel comprend une boucle de régulation LIC qui contrôle le débit de reflux. Le débit vapeur total arrivant au bouilleur est piloté par asservissement de la charge thermique au bouilleur à la perte de charge de la colonne (*ΔP = 3 mbar*)*.* Le condenseur est un tubes-et-calandre horizontal comprenant 80 tubes ; de DN10 (DN : Diamètre Nominal en mm) et de longueur 1150 mm.
Le frigoporteur est une huile Gilotherm à 85°C et au débit de 533 L/h.
La hauteur de liquide de consigne du LIC est de 165 mm. Cette émergence permet d'obtenir 44 kg/h d'évents pour 176 kg/h de phase vapeur qui entre dans le condenseur. La soupape tarée de l'évent du condenseur est réglé à 5 mbar (ouverture si > 5 mbar). Le débit de vapeur vive à l'entrée de la colonne (6) est régulé de façon à assurer une perte de charge de 3,3 mbar.

### Exemple 4 : Distillation du flux (F1) sur colonne à cloison avec soutirage en phase vapeur

L'exemple 4 fait référence à la figure 3.
Le flux *(F1)* est alimenté au débit de 200 kg/heure en tête d'un échangeur à film tombant (1). Cet échangeur permet d'éliminer l'acétate d'éthyle sous un flux *(F11)* au débit de 147 kg/heure. Le flux *(F11)* est envoyé vers un condenseur (non représenté) pour récupération de l'acétate d'éthyle.
Un flux *(F2)* est récupéré en sortie de l'évaporateur qui correspond au flux *(F1)* substantiellement exempt d'acétate d'éthyle. Les traces d'acétate d'éthyle présentes dans le flux *(F2)* sont récupérées dans le piège à froid d'une colonne de distillation (7) permettant ainsi de protéger la pompe de la colonne. Le flux *(F2)* est envoyé vers une colonne de distillation (7) au débit de 53 kg/heure. On récupère :
   - un flux *(F32)* comprenant les légers et 3% en poids de vanilline à un débit de 5 kg/heure en tête de colonne ;
   - un flux *(F33)* en soutirage latéral comprenant la vanilline avec une pureté de 98,9% et 1,1% en poids de lourds essentiellement constitué d'alcool vanillique à un débit de 39 kg/heure. Le rendement du procédé de purification est égal à 96.5% ;
   - un flux *(F34)* en pied de colonne comprenant les lourds et 3% en poids de vanilline à un débit de 8 kg/heure.

Les caractéristiques du dispositif sont les suivantes :
- Echangeur (1) : Surface = 0,5 m2.
- Colonne (7)

Colonne à cloison équipée d'un segment de cloisonnement (8) délimitant une demi-colonne primaire (71) et une demi-colonne secondaire (72)
- un garnissage ordonné de surface spécifique 450 m2/m3 dans la demi colonne primaire (71) sur une hauteur de 5410 mm et d'un garnissage ordonné dans la demi-colonne secondaire (72) sur une hauteur de 4560 mm

La demi-colonne primaire a un nombre d'étages de 27
La demi-colonne secondaire a un nombre d'étages de 23
La pression de service : 4 mbar
Le taux de reflux : 3 dans chaque demi-colonne.

Un passage (9) dans le segment (8) à la cote 3110 mm permet d'enrichir la section de concentration de la 2^{ème} demi-colonne. L'ouverture du passage est une bande qui n'excède pas 10 mm de large

Une différence de température entre pied et tête de 74.9°C quand la température de tête avoisine 116°C.

Le débit de reflux de la section aval représente 2,18 fois le débit d'alimentation *(F2)* et celui de la section amont représente 25 % du débit d'alimentation *(F2).*

La perte de charge de consigne est de 1,3 mbar pour la colonne.

L'énergie pour la distillation est apportée par un bouilleur de type film raclé ou un tubes-et-calandre à circulation forcée.

### Exemple 5 : Distillation du flux (F1) sur colonne à cloison par double soutirage vapeur/liquide

L'exemple 5 fait référence à la figure 3 à laquelle est ajouté un point de soutirage liquide supplémentaire.
Le flux *(F1)* est alimenté au débit de 200 kg/heure en tête d'un échangeur à film tombant (1). Cet échangeur permet d'éliminer l'acétate d'éthyle sous un flux *(F11)* au débit de 147 kg/heure. Le flux *(F11)* est envoyé vers un condenseur pour récupération de l'acétate d'éthyle.
Un flux *(F2)* est récupéré en sortie de l'évaporateur qui correspond au flux *(F1)* substantiellement exempt d'acétate d'éthyle. Les traces d'acétate d'éthyle présentes dans le flux *(F2)* sont récupérées dans le piège à froid d'une colonne de distillation (7) permettant ainsi de protéger la pompe de la colonne. Le flux *(F2)* est envoyé vers une colonne de distillation (7) au débit de 53 kg/heure. On récupère :
   - un flux *(F32)* comprenant les légers et 3% en poids de vanilline à un débit de 5 kg/heure en tête de colonne ;
   - un flux *(F33)* en soutirage latéral en phase vapeur comprenant la vanilline avec une pureté de 99,8% et 0,2% en poids de lourds essentiellement constitué d'alcool vanillique à un débit de 38,2 kg/heure ;
   - un flux (F35) (non représenté) en soutirage latéral en phase liquide comprenant la vanilline avec une pureté de 98% et 2% en poids de lourds à un débit de 1,7 kg/heure ;
   - un flux *(F34)* en pied de colonne comprenant les lourds et 3% en poids de vanilline à un débit de 8 kg/heure.
Le rendement global de la purification est de 98,5%.

Les caractéristiques du dispositif sont les suivantes :
- Echangeur (1) : Surface = 0,5 m2.
- Colonne (7)

Colonne à cloison équipée d'un segment de cloisonnement (8) délimitant une demi-colonne primaire (71) et une demi-colonne secondaire (72)
- un garnissage ordonné de surface spécifique 450 m2/m3 dans la demi colonne primaire (71) sur une hauteur de 5410 mm et d'un garnissage ordonné dans la demi-colonne secondaire (72) sur une hauteur de 4560 mm

La demi-colonne primaire a un nombre d'étages de 27
La demi-colonne secondaire a un nombre d'étages de 23
La pression de service : 4 mbar
Le taux de reflux : 3 dans chaque demi-colonne.

Un passage (9) dans le segment (8) à la cote 3110 mm permet d'enrichir la section de concentration de la 2^{ème} demi-colonne. L'ouverture du passage est une bande qui n'excède pas 10 mm de large

Le soutirage latéral en phase vapeur est situé à 1200 mm par rapport à la base du lit de garnissage.

Le soutirage latéral en phase liquide est situé 400 mm en-dessous du soutirage latéral en phase vapeur.

Une différence de température entre pied et tête de 74.9°C quand la température de tête avoisine 116°C.

Le débit de reflux de la section aval représente 2,18 fois le débit d'alimentation *(F2)* et celui de la section amont représente 25 % du débit d'alimentation *(F2).*

La perte de charge de consigne est de 1,3 mbar pour la colonne.

L'énergie pour la distillation est apportée par un bouilleur de type film raclé ou un tubes-et-calandre à circulation forcée.

### Exemple 6 - Purification dans un évaporateur à film-raclé (selon l'invention) :

10 kg de vanilline naturelle, présentant une pureté de 98,5% en poids et une coloration d'environ 5 Gardner (selon la norme ASTM D1544) en solution éthanolique à 10% en poids, sont fondus dans une étuve, sous azote, à 95°C et à pression atmosphérique. La vanilline naturelle fondue est ensuite ajoutée dans une ampoule de coulée thermostatée qui alimente un évaporateur à film raclé court-trajet de 0,05 m2 (évaporateur KDL-5 commercialisé par la société UIC GmbH) à une température de 135°C et sous une pression de 3 mbar. La vitesse de rotation de l'évaporateur est de 200 tours/min et le temps de séjour de la vanilline dans l'évaporateur est de 45 secondes. Le débit d'alimentation utilisé est de 500g/h de vanilline naturelle fondue.

Les vapeurs de vanilline naturelle sont condensées à 100°C sur le condenseur interne de l'évaporateur à film raclé et le condensat liquide est dirigé vers une bande de pastillage refroidie à 10°C. Le résidu comprenant des oligomères phénoliques est évacué dans un réceptacle à résidus.

Les pastilles sont récupérées puis pesées.

A l'issue du procédé selon l'invention, 9,8 kg de vanilline naturelle purifiée sont obtenus, soit un rendement de 98% en poids, présentant une pureté supérieure ou égale à 99,5% en poids, et de couleur (en solution éthanolique à 10% en poids) égale à 60 Hazen (norme ISO 6271).

### Exemple 7 (comparatif):

Le même lot de vanilline naturelle quà l'exemple 6, présentant une pureté de 98,5% en poids, a été purifié par recristallisation dans un mélange eau/éthanol (20/80 en poids). A l'issue de la purification par recristallisation, de la vanilline naturelle purifiée, présentant un degré de pureté égal à 98,8% en poids, est obtenue avec un rendement de 80% en poids. En solution éthanolique à 10% en poids, elle présente une coloration de 4 Gardner (selon la norme ASTM D1544), c'est-à-dire très supérieure à 500 Hazen.

En définitive, le procédé selon l'invention permet d'obtenir de la vanilline naturelle très pure, avec un meilleur rendement, et surtout une coloration beaucoup plus faible (vanilline beaucoup plus blanche).

## Revendications

1. Utilisation d'un évaporateur à film tombant ou d'un évaporateur à film raclé pour la séparation de la vanilline naturelle et des lourds.

2. Utilisation d'un évaporateur à film tombant ou d'un évaporateur à film raclé pour la préparation d'une vanilline naturelle ayant une pureté supérieure ou égale à 99% en poids et faiblement coloré.

3. Utilisation selon la revendication 1 ou 2 pour la préparation d'une vanilline naturelle ayant une couleur inférieure ou égale à 200 Hazen en solution éthanolique à 10% en poids.

4. Procédé de préparation de vanilline naturelle comprenant l'utilisation selon l'une quelconque des revendications 1 à 3.

5. Procédé de préparation de vanilline naturelle comprenant une étape de distillation permettant de récupérer en tête de distillation un flux (F14) comprenant la vanilline naturelle, ou un flux (F22) comprenant les légers et la vanilline naturelle, et en pied de distillation un flux (F15 ou F23) comprenant les lourds.

6. Procédé selon la revendication 5 dans lequel l'apport des calories en pied de colonne dans ladite étape de distillation est faite dans un bouilleur à film tombant ou à film raclé, par un échangeur tubes-calandre chauffé à la vapeur ou par un fluide caloporteur, par l'intermédiaire de serpentins de chauffage alimentés à la vapeur ou par un fluide caloporteur.

7. Procédé selon la revendication 5 ou 6 dans lequel l'apport des calories en pied de colonne dans ladite étape de distillation est faite dans un bouilleur à film tombant un tubes-et-calandre à circulation forcée ou un tubes-et-calandre à thermosiphon.

8. Procédé selon la revendication 5 ou 6 dans lequel l'apport des calories en pied de colonne dans ladite étape de distillation est faite dans un échangeur raclé ou un tubes-et-calandre à circulation forcée.
